# EUROPEAN PATENT SPECIFICATION

(11) **EP 2 155 886 B1**
(45) Date of publication and mention of the grant of the patent: **07.03.2012**
(21) Application number: 08754745.1
(22) Date of filing: 27.05.2008
(51) Int. Cl.: A61Q 19/00, A61K 8/37

(54) **Cosmetic use of inhibitors of tyrosinase**
Kosmetische Verwendung von Inhibitoren von Tyrosinase
Utilisation cosmétique d'inhibiteurs de tyrosinase

(30) Priority: 08.06.2007 US 811055
(43) Date of publication of application: 24.02.2010
(60) Divisional application: 11009584.1
(73) Proprietor: EASTMAN CHEMICAL COMPANY, Kingsport TN 37660 (US)
(72) Inventor: CLENDENNEN, Stephanie Kay, Kingsport, TN 37663 (US); BOAZ, Neil Warren, Kindsport, TN 37660 (US); CLAUSON, Jeffrey, Michael, Johnson City, TN 37604 (US)
(74) Representative: Bradley, Adrian
(86) International application number: PCT/US2008/006699
(87) International publication number: WO 2008/153784

(56) References cited:
- JP-A- 2003 155 283
- US-A- 4 369 174
- LIU ET AL: "Lipase-catalyzed synthesis of kojic acid esters in organic solvents" JOURNAL OF THE AMERICAN OIL CHEMISTS' SOCIETY, vol. 75, 1998, pages 1507-1511, XP002512109 cited in the application
- ADACHI ET AL: "Synthesis of esters by immobilized-lipase-catalyzed condensation reaction of sugars and fatty acids in water-miscible organic solvent" JOURNAL OF BIOSCIENCE AND BIOENGINEERING, vol. 99, 2005, pages 87-94, XP004922398
- RAKU ET AL: "Regioselective synthesis of kojic acid esters by Bacillus subtilis protease" BIOTECHNOLOGY LETTERS, vol. 25, 2003, pages 969-974, XP002512244
- N.N.: "Process development of kojic acid ester production by using enzyme" NATIONAL TSING HUA UNIVERSITY REPOSITORY, 2005, page 1, XP002512245 Retrieved from the Internet: URL:140.114.72.28/handle/987654321/6199> [retrieved on 2009-01-27]
- KHAMARUDDIN ET AL: "Enzymatic synthesis and characterization of palm-based kojic acid ester" JOURNAL OF OIL PALM RESEARCH, vol. 20, June 2008 (2008-06), pages 461-469, XP002512246

## Description

### FIELD OF THE INVENTION

The present invention relates to a cosmetic method of brightening skin comprising the application of compositions based on esters of substituted methanols which reduce melanin formation.

### BACKGROUND OF THE INVENTION

Hyperpigmentation of the skin is directly related to the formation of melanin, a dark pigment formed from tyrosine. The initial steps in the conversion of tyrosine to melanin arc mediated by the enzyme tyrosinase. Effective inhibitors of tyrosinase can inhibit melanin formation and are useful for reducing undesirable pigmentation of the skin (e.g. skin brightening, evening out skin tone or reducing the appearance of age spots). There arc currently several tyrosinase inhibitors in the marketplace, including hydroquinone, kojic acid and arbutin. However, there arc disadvantages for each of these products. For example, kojic acid displays low bioavailability and thus marginal efficacy. Another example, hydroquinone, is oxidized by air, light and tyrosinase itself. These oxidized products of hydroquinone have been implicated in skin irritation and perhaps cytotoxicity.

Kojic acid is commonly used as a skin brightening ingredient. It is a fungal metabolic product that has been show to be both sale and effective for topical use (reviewed in Burdock et al., 2001, Regulatory Toxicology and Pharmacology 33: 80-101). Monoeslers and diesters of Kojic acid have also been described (Nagai, S.; Izumi, T., U.S. Pat. No. 4,369,174) and appeal to have excellent tyrosinase-inhibiting activity so as to inhibit melanin formation in the skin. This inhibition can produce excellent effects in brightening of the skin.

There are two distinct monoesters that can be prepared from kojic acid or 4-hydroxybenzyl alcohol, as the parent molecules have both an enol alcohol (or a phenol) and a primary alcohol. The monoesters of kojic acid reported in U.S. Pat. No. 4,369,174 were prepared by chemical methods under high temperature conditions. and afforded the ester of the primary alcohol. The drastic conditions utilized would not be feasible for thermally unstable reaction partners. Kojic acid has been reported to be acylated enzymatically specifically on the enol oxygen (Liu, K.-J; Shaw, J.-F. J. Am. Oil Chem. Soc. 1998, 75, 1 507-1511). In a conflicting report, a Japanese patent application (application number 2002-257704) indicated that kojic acid was enzymatically acylated on the hydroxymethyl oxygen when using an acid or vinyl ester as the acylating agent.

Inhibitors that are more bioavailable and efficacious present enhanced potential for a noticeable brightening benefit with a lack of skin irritation. Other likely benefits will include ease of use, improved shelf life and decreased frequency of application. It is the object of this invention to provide a cosmetic method of skin brightening using such compounds and composition.

### SUMMARY OF THE INVENTION

The invention provides a cosmetic method of brightening skin comprising the application to a skin site, of a composition comprising an ester compound represented by formula 1: 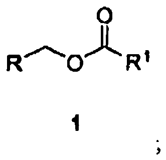 and
a cosmetically acceptable carrier
wherein R is selected from the group consisting of substituted or unsubstituted C₆-C₂₂ carbocyclic hydroxyaryl and
R¹ is selected from the group consisting or C₁-C₂₂ alkyl, C₂-C₂₂ alkenyl, C₄-C₂₂ dienyl, C₆-C₂₂ trienyl, C₈-C₂₂ tetraenyl and mixtures thereof.

### DETAILED DESCRIPTION

Further disclosed is a mild, simple, biocatalytic method for the preparation of monomers and related materials wherein the ester is formed a primary oxygen instead of at an enol oxygen. These compounds function as highly effective tyrosinase inhibitors.

A method for the preparation of ester compounds represented by the general formula (x) is disclosed: 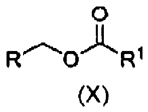 wherein
R is selected from substituted or unsubstituted C₆-C₂₂ carbocyclic hydroxyaryl, aubstitutcd hydroxy-4H-pyran-4-on-2-yl and substituted or unsubstituted C₁-C₂₀) hydroxyheteroaryl wherein the heteroatoms are selected from sulfur, nitrogen, and oxygen; and
R¹ is selected from substituted and unsubstituted, branched- and straight-chain saturated, C₁-C₂₂ alkyl, substituted and unsubstituted, branched- and straight-chain C₂-C₂₂ alkenyl, substituted and unsubstituted, branched- and straight-chain C₄-C₂₂ dienyl, substituted and unsubstituted, branched- and straight-chain C₆-C₂₂ trienyl, and substituted and unsubstituted, branched- and straight-chain C₈-C₂₂ tetraenyl or mixtures thereof.

The aryl groups which R may represent may include phenyl, naphthyl, or anthracenyl and phenyl, naphthyl, or anthracenyl substituted with a hydroxyl group and one to three additional substituents selected from C₁-C₆-alkyl, substituted C₁-C₆-alkyl, C₆-C₁₀ aryl, substituted C₆-C₁₀ aryl, C₁-C₆-alkoxy, hydroxy, halogen, carboxy, cyano, C₁-C₆-alkanoyloxy, C₁-C₆-alkylthio, C₁-C₆-alkylsulfonyl, trifluoromethyl, hydroxy, C₂-C₆-alkoxvcarbonyl, C₂-C₆-alkanoylamino and -O-R², S-R², -SO₂-R², - NHSO₂R² and -NHCO₂R², wherein R² is phenyl, naphthyl, or phenyl or naphthly substituted with one to three groups selected from C₁-C₆-alkyl, C₆-C₁₀ aryl, C₁-C₆alkoxy and halogen.

The heteroaryl groups which R may represent include a 5- or 6- membered hydroxy-substitited aromatic ring containing one to three heteroatoms selected from oxygen, sulfur and nitrogen. Examples of such heteroaryl groups are hydroxythienyl, hydroxyfuryl, hydroxypyrrolyl, hydroxyimidazolyl, hydroxypyrazolyl, hydroxythiazolyl, hydroxyisothiazolyl, hydroxyoxazolyl, hydroxyisoxazolyl, hydroxytriazolyl, hydroxythiadiazolyl, hydroxyoxadiazolyl, hydroxytetrazolyl, hydroxypyridyl, hydroxypyrimidyl, hydroxybenzoxazolyi, hydroxybenzothiazolyl, hydroxy benzimidazolyl, hydroxyindolyl and the like. The heteroaryl radicals may be substituted, for example, with up to three additional groups such as C₁-C₆-alkyl, C₁-C₆-alkoxy, substituted C₁-C₆-alkyl, hydroxy, halogen, C₁-C₆-alkylthio, aryl, arylthio, aryloxy, C₂-C₆-alkoxycarbonyl and C₂-C₆-alkanoylamino. The heteroaryl radicals also may be substituted with a fused ring system, e.g., a benzo or naphtho residue, which may be unsubstituted or substituted, for example, with up to three of the groups set forth in the preceding sentence.

The term "halogen" is used to include fluorine, chlorine, bromine, and iodine.

The alkyl, alkenyl, dienyl, trienyl, and tetraenyl groups which may be represented by R¹ may be straight- or branched-chain aliphatic hydrocarbon radicals containing up to about 20 carbon atoms and may be substituted, for example, with one to three groups selected from C₁-C₆-alkoxy, cyano, C₂-C₆-alkoxycarbonyl, C₂-C₆-alkanoyloxy, hydroxy, aryl, heteroaryl, thiol, thioether, dithiolane, and halogen. The terms "C₁-C₆-alkoxy", "C₂-C₆-alkoxycarbonyl", and "C₂-C₆-alkanoyloxy" are used to denote radicals corresponding to the structures -OR³, -CO₂R³, and -OCOR³, respectively, wherein R³ is C₁-C₆-alkyl or substituted C₁-C₆-alkyl.

The compounds for use in the cosmetic method of the invention are defined in formula **1**, and the preferred ones are those in which R is phenol.

Particularly preferred compounds are denoted by structures **1** wherein R is 4-hydroxyphenyl and R¹ is selected from C₁-C₁₆ linear alkyl groups.

The preparation process comprises the reaction of alcohol **2**: 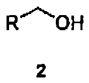 with an acid derivative of formula 3: 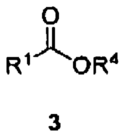 in the presence of a lipase and molecular sieves, and in the presence or absence of an organic solvent to form the desired ester **1** wherein the substituent R of the alcohol **2** and R¹ of acid derivative **3** are as defined above und substituent R⁴ of the acid derivative is chosen from among hydrogen and C₁-C₄ substituted or unsubstituted alkyl groups. Examples of the C₁-C₄ alkyl groups include methyl, ethyl, n-propyl, i-propyl, n-butyl, i-butyl, s-butyl and the like. Preferred substituents R¹ include hydrogen, methyl any ethyl, with hydrogen the most preferred.

The process may be carried out without additional solvent or optionally in an inert solvent chosen from cyclic or cyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichlorocthane, dibromoethane, tetrachloroethylene, or chlorobenzene, polar aprotic solvents such as acetonitrile, dimethyl formamide, or dimethyl sulfoxide, or mixtures thereof. The preferred solvents are toluene and acetonitrile. The process may be carried out at a temperature between about -100°C. and the boiling point of the solvent, preferably about 0-70°C., most preferably 20-60°C. The amount of the acid derivative **3** may be between 0.85 and 20 equivalents based on **2**, and is preferably between 1 and 10 equivalents. The enzyme used in the process is a lipase. The lipase may be in the form of whole cells, isolated native enzymes, or immobilized on supports. Examples of the lipase include but are not limited to Lipase PS (from *Pseudomonas sp*), Lipase PS-C (from *Psuedomonas sp* immobilized on ceramic), Lipase PS-D (from *Pseudomonas sp* immobilized on diatomaceous earth), Lipoprime 50T, Lipozyime TL IM, or Navozyme 435 (from *Candida antarctica* immobilized on acrylic resin).

The process may optionally be carried out in the presence of various addenda chosen from molecular sieves or ion exchange resins. Particularly preferred are molecular sieves, as the presence of these materials can remove byproducts such as water or lower chain alcohols generated during the reaction. Examples of these include 3A, 4A, and 5A molecular sieves.

The product of the process may he isolated using methods known to those of skill in the art, e.g., extraction, filtration, or crystallization. The product 1 may be purified if necessary using methods known to those of skill in the art, e.g., extraction, chromatography, distillation, or crystallization.

Another disclosed process involves the transesterification of an ester **4**: 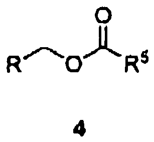 with an acid derivative of formula **3**: 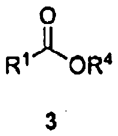 in the presence of a lipase and in the presence or absence of an organic solvent to form the desired ester **1** wherein the substituent R of the ester **4** and R¹ and R⁴ of acid derivative **3** arc as defined above and substituent R⁵ of ester **4** is chosen from among hydrogen and C₁-C₄ substituted or unsubstituted alkyl groups. Examples of the C₁-C₄ alkyl groups include methyl, ethyl, n-propyl, i-propyl, n-butyl, 1-butyl, s-butyt and the like. Preferred substituents R⁵ include hydrogen, methyl, and ethyl.

The process may be earned out without additional solvent or optionally in an inert solvent chosen from cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acctonitrile, dimethyl formamide, or dimethyl sulfoxide, or mixtures thereof. The preferred solvents arc toluene and acetonitrile. The process may be carried out at a temperature between about -100°C and the boiling point of the solvent, preferably about 0-70°C, most preferably 20-60°C. The amount of the acid derivative **3** may be between 0.85 and 20 equivalents based on **4**, and is preferably between 1 and 10 equivalents. The enzyme used in the process is a lipase. The lipase may be in the form of whole cells, isolated native enzymes, or immobilized on supports. Examples of the lipase include but are not limited to Lipase PS (from *Pseudomonas sp* Lipase PS-C (from *Psuedomonas sp* immobilized on ceramic), Lipase PS-D (from *Pseudomonas sp* immobilized on diatomaceous earth), Lipoprime 50T, Lipozyme TL IM, or Novozym 435 (from *Candida antarctica* immobilized on acrylic resin).

The process may optionally be carried out in the presence of various addenda chosen from molecular sieves or ion exchange resins. Particularly preferred are ion exchange resins. Examples of these resins are Amberlite^{R} or Amberlyst^{R} weakly basic resins, such as Amberlite IRA-95, Amberlite IRA-94, and Amberlyst A-21, although it appears that any weakly basic resin will be acceptable.

The product of the process may be isolated using methods known to those of skill in the art, e.g., extraction, filtration, or crystallization. The product 1 may be purified if necessary using methods known to those of skill in the art, e.g., extraction, chromatography, distillation, or crystallization.

Another disclosed process involves the reaction of alcohol **2**: 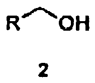 with an acid anhydride of formula **5**: 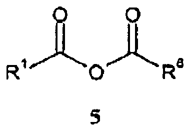 in the presence of a lipase and in the presence or absence of an organic solvent to form the desired ester **1** wherein the substituent R of the alcohol **2** and R¹ of acid anhydride **5** are as defined above and substituent R⁶ of the acid anhydride is selected from substituted and unsubstituted, branched- and straight-chain saturated, C₁-C₂₂ alkyl, substituted and unsubstituted, branched- and straight-chain C₂-C₂₂ alkenyl, substituted and unsubstituted, branched- and straight-chain C₄-C₂₂ dienyl, substituted and unsubstituted, blanched- and straight-chain C₆-C₂₂ trienyl, and substituted and unsubstituted, branched- and straight-chain C₈-C₂₂ tetraenyl or mixtures thereof. Preferred acid anhydrides include those wherein R¹ and R⁶ arc identical.

The process may be carried out without additional solvent or optionally in an inert solvent chosen from cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acetonitrile or dimethyl formamide or mixtures thereof. The preferred solvents are toluene and acetonitrile. The process may be carried out at a temperature between about -100°C and the boiling point of the solvent, preferably about 0-70°C, most preferably 20-60°C. The amount or the acid anhydride may be between 0.85 and 20 equivalents, based on **2**, and is preferably between 1 and 5 equivalents. The enzyme used in the process is a lipase. The lipase may he in the form of whole cells, isolated native enzymes, or immobilized on supports. Examples of the lipase include but are not limited to Lipase PS (from *Pseudomonas sp*), Lipase PS-C (from *Psuedomonas sp* immobilized on ceramic), Lipase PS-D (from *Pseudomonas sp* immobilized on diatomaccous earth), Lipoprime 50T, Lipozyme TL IM, or Novozyme 435 (from *Candida antarctica* immobilized on acrylic resin).

The product of the process may be isolated using methods known to those of skill in the art, e.g., extraction, filtration, or crystallization. The product **1** may be purified if necessary using methods known to those of skill in the art, e.g., extraction, chromatography, distillation, or crystallisation.

The early steps of melanin biosynthesis from tyrosine in mammalian skin is catalyzed by the enzyme tyrosinase. Compounds that inhibit tyrosinase are effective at reducing skin pigmentation. The ability of a compound to reduce skin pigmentation can be very effectively predicted by measuring the tyrosinase inhibiting activity in an *in vitro* assay. A purified tyrosinase (usually from mushroom) is incubated in the presence of a tyrosinase substrate (L-DOPA) and varying concentrations of the compound to be tested. The concentration-dependent activity of the test compound is measured as the degree of inhibition of the tyrosinase-catalyzed oxidation of L-DOPA, a colorimetric reaction. Other methods for testing the activity of a skin-brightening compound include: Exposing cultured primary or immortalized melanocyte cell culture (often murine or human-derived) to the compounds and measuring melanin production; exposing a reconstructed skin model containing co-cultured melanocytes, keratinocytes and/or fibroblasts; or applying the compound to the skin of a mammalian subject, while monitoring chances in surface color or reflectance over time (e.g. Virador et al. Analytical Biochemistry 1999, 270, 207; Boissy et al. Experimental Dematology 2005, 14, 601).

The tyrosinase inhibition assay is a well-accepted method for measuring the potential skin-brightening activity of a test compound (e.g. Um et all. Bioorganic & Medicinial Chemistry 2003, 11, 5345). The esters of this invention show potent ability to inhibit the enzyme tyrosinase.

Typical skin brightening compositions for use in the cosmetic method of the invention contain at least 0.0001 % by weight of the esters disclosed. For example, the compositions can contain from about 0.0001 % to about 10.0 % by weight or from about 0.0001 % to about 2.0 % by weight of the esters disclosed. Lower concentrations may be employed for less pronounced hyperpigmentation conditions and in sunscreens and sunblocks used after skin brightening treatment, and higher concentrations may be employed with more acute pigmentation conditions. Suggested ranges also depend upon any adjunct ingredients employed in the compositions and the user's colouring and skin type as well as the extent or severity of the hyperpigmentation problem.

The skin brightening compositions may also contain other skin brightening ingredients in addition to esters. Such other ingredients are known to those of skill in the are.

Typically, topical application to skin sites is accomplished in association with a carrier. Where employed, the carrier is inert in the sense or not bringing about a deactivation or oxidation of active or adjunct ingredient(s), and in the sense of not bringing about any adverse effect on the skin areas to which it is applied. For example, the compounds according to the present invention are applied in admixture with a dermatolgically acceptable carrier or vehicle (e.g., as a lotion, cream, ointment, soap, stick, or the like) so as to facilitate topical application and, in some cases, provide additional beneficial effects as might he brought about, e.g., by moisturizing of the affected skin areas. Many preparations are known in the art, and include lotions containing oils and/or alcohols and emollients such as olive oil, hydrocarbon oils and waxes, silicone oils, other vegetable, animal or marine fats or oils, glyceride derivatives, fatty acids or fatty acid esters or alcohols or alcohol ethers, lecithin, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophilic colloids.

### REFERENCE EXAMPLES

The processes disclosed arc further illustrated by the following reference examples.

### Reference Example 1

### Preparation of 5-hydroxy-4H-pyran-4-on-2-methyl acetate (1a)

2-Hydroxymethyl-5-hydroxy-4H-pyran-4-one (**2a**; 500 mg; 3.52 mmol) was slurried in 10 mL of acetonitrile. vinyl acetate (324 uL; 3.52 mmol; 1.0 equiv) was added, followed by 120 mg of Novozyme 435. The mixture was heated to 50°C. for 6 h, at which point tle analysis indicates significant conversion to **1a**. An additional 0.2 equiv of vinyl acetate was added, and the mixture was heated overnight to afford a small amount of residual **2a.** An additional 0.2 equiv of vinyl acetate was added, and the mixture was heated at 50°C for 12 h to completely consume **2a** according to tle analysis. The mixture was filtered while warm to remove the enzyme and the filtrate was stripped to afford 0.64 g (99%) of **1a**. This compound was a potent inhibitor of tyrosinase (EC₅₀ 0.0049 mM), significantly better than **2a** (EC₅₀ 0.015 mM)(scc Table 1).
¹H NMR (DMSO-d₆) δ 9.24 (br s, I H); 8.08 (s, 1H); 6.47 (s, 1H); 4.93 (s, 2H); 2. 10 (s, 3H).

### Reference Example 1'

### Preparation of 2-hydroxymethyl-4H-pyran-4-on-5-yl acetate (6a)

2-Hydroxymethyl-5-hydroxy-4H-pyran-4-one (**2a**; 1.00 g, 7.04 mmol) was slurried in 30 mL of dichloromethane. Triethylamine (1.47 mL; 10.6 mmol; 1.5 equiv) was added und acetic anhydride (0.69 mL; 7.04 mmol; 1.0 equiv) was then added dropwise. The heterogeneous mixture became homogeneous over 30 min and was stirred overnight to afford a major spot of intermediate polarity by tle analysis (ethyl acetate cluant) as well as a non-polar spot. The reaction mixture was concentrated and filtered through a pad of flash silica gel and eluted with a solvent gradient of 4:1 ethyl acetate:heptane to 100% ethyl acetate. A center cut of the intermediate spot was collected, affording 415 mg (32%) of 6a. This compound was a significantly less potent inhibitor of tyrosinase (EC₅₀ 0.084 mM) than **2a** (EC₅₀ 0.015 mM)(see Table 1).
¹H NMR (DMSO-d₆) δ 8.46 (s, 1H); 6.42 (s, 1H); 5.77 (br s, 1H); 4.34 (br s, 2H); 2.25 (s, 3H).

### Reference Example 2

### Preparation of 5-hydroxy-4H-pyran-4-on-2-methyl propionate (1b)

Novozyme 435 (120 mg) and dried 4A molecular sieves (1 g) were added to a 50-mL flask, 2-Hydroxymethyl-5-hydroxy-4H-pyran-4-one (**2a**; 500 mg; 3.52 mmol) was added and washed in with 10 mL of acetonitrile. Propionic acid (525 uL; 7.04 mmol; 2 equiv) was added and the mixture was heated to 50°C overnight at which point tic analysis (ethyl acetate oluent) indicated conversion to **1b**. The reaction mixture was filtered and the filtrate was concentrated at reduced pressure. The crude product was filtered through a pad of flash silica gel and eluted with 4:1 ethyl acetate:heptane to afford 285 mg (41%) of **1h**. This compound was a potent inhibitor of tyrosinase (EC₅₀ 0.0046 mM), significantly better than **2a** (EC₅₀ 0.015 mM)(see Table 1).
¹H NMR (DMSO-d₆) δ 9.25 (br s, 1H); 8.09 (s, 1H); 6.46 (s, 1H); 4.95 (s, 2H); 2.42 (q, 2H, J = 7.42 Hz); 1.04 (t, 3H, J = 7.42 Hz).

### Reference Example 3

### Preparation of 5-hydroxy-4H-pyran-4-on-2-methyl propionate (1b) by enzymatic esterification using propionic anhydride

2-Hydroxymethyl-5-hydroxy-4H-pyran-4-one (**2a**; 500 mg; 3.52 mmol) was slurried in 10 mL of acetonitrile and propionic anhydride (0.54 mL; 4.2 mmol; 1.2 equiv) was added. Novozyme 435 (120 mg) was added and the mixture was stirred at ambient temperature for 5.5 h to almost completely convert **2a** to **1b** according to HPLC and tlc analysis (ethyl acetate eluent). The reaction mixture was filtered and the filtrate was concentrated at reduced pressure. The crude product was dissolved in ethyl acetate and diluted with heptane to afford a precipitate, which was collected, washed with heptane, and dried. This filtrate was concentrated to dryness and the resulting solid was triturated with heptane, filtered, washed with heptane, and dried. The combined solids (576 mg; 83%) analyzed as pure **1b** by ¹H NMR.

### Reference Example 2'

### Preparation of 2-hydroxymethyl-4H-pyran-4-on-5-yl propionate (6b)

2-Hydroxymethyl-5-hydroxy-4H-pyran-4-one (**2a**; 852 mg; 6.00 mmol) was slurried in 30 mL of dichloromethane. Triethylamine (1.25 mL; 9.0 mmol; 1.5 equiv) was added and then propionic anhydride (0.77 mL; 6.0 mmol; 1.0 equiv) was added dropwise at ambient temperature. The reaction mixtures became homogeneous within one minute and was stirred overnight to afford one major spot by tlc (ethyl acetate eluant). The volatiles were stripped and the residue was dilulted with ethyl acetate and washed with 1 M HCl (15 mL) and saturated sodium bicarbonate (15 mL). The organic solution was dried with magnesium sulfate and concentrated to afford 0.92 g of crude product. Analysis of the crude product by ¹H NMR indicated a small amount of **2a**, a small amount of the dipropionate of **2a** (**7b**), and a 90:10 ratio of **6b** to **1b**. The mixture was filtered through a pad of flash silica gel and eluted with 1:1 ethyl acetate:heptane to 4:1 ethyl acetate:heptane to afford 476 mg (40%) of **6b**. This compound was a significantly less potent inhibitor of tyrosinase (EC₅₀ 0.10 mM) than **2a** (EC₅₀ 0.015 mM)(see Table 1).
¹H NMR (DMSO-d₆) δ 8.46 (s, 1H); 6.42 (t, 1H, J = 0.82 Hz); 5.76 (t, 1H, J = 6.05 Hz); 4.34 (dd, 2H; J = 0.82, 6.05 Hz); 2.58 (q, 2H, J = 7.42 Hz); 1.11 (t, 3H, J = 7.42 Hz).

### Reference Example 3'

### Preparation of 2-propionyloxymethyl-4H-pyran-4-on-5-yl propionate (7b)

2-Hydroxymethyl-5-hydroxy-4H-pyran-4-one (**2a**; 852 mg; 6.00 mmol) was slurried in 30 mL of dichloromethane. Triethylamine (2.51 mL; 18.0 mmol; 3 equiv) was added and then propionic anhydride (1.69 mL; 13.2 mmol; 2.2 equiv) was added dropwise at ambient temperature. The reaction mixture became homogeneous within one minute and was stirred overnight to afford one major spot by tic (ethyl acetate eluant). The volatiles were stripped and the residue was diluted with ethyl acetate and washed with I M HCl (15 mL) and saturated sodium bicarbonate (15 mL). The organic solution was dried with magnesium sulfate and concentrated to afford 2.30 g of crude product, which was filtered through a pad of flash silica gel and eluted with 1:1 methyl acetate:heptane to afford 1.39 g (97%) of **7b**. This compound was a significantly less potent inhibitor of tyrosinase (EC₅₀ 0.097 mM) than **2a** (EC₅₀ 0.015 mM)(see Table 1).
¹H NMR (DMSO-d₆) δ 8.52 (s, 1H); 6.59 (s, 1H); 5.01 (s, 1H); 2.59 (q, 2H; J = 7.42 Hz); 2.44 (q, 2H, J = 7.42 Hz); 1.11 (t, 3H, J = 7.42 Hz); 1.05 (t, 3H, J = 7.42 Hz).

### Reference Example 4

### Preparation of 5-hydroxy-4H-pyran-4-on-2-methyl hexanoate (1c) by enzymatic esterification using hexanoic anhydride

2-Hydroxymethyl-5-hydroxy-4H-pyran-4-one (**2a**; 500 mg; 3.52 mmol) was slurried in 10 mL of acetonitrile and hexanoic anhydride (0.81 mL; 3.52 mmol; 1.0 equiv) was added. Novozyme 435 (120 mg) was added and the mixture was stirred at ambient temperature overnight to afford 85% conversion of **2a** to **1c** according to HPLC analysis. Additional hexanoic anhydride (0.12 mL; 0.53 mmol; 0.15 equiv) was added and the mixture was stirred overnight to afford 98% conversion of **2a** to **1c**. The reaction mixture was filtered and the filtrate was concentrated at reduced pressure at ambient temperature. The residue was dissolved in ethyl acetate and washed with water (10 mL) and saturated aqueous sodium bicarbonate solution (3x10 mL). The organic solution was dried with magnesium sulfate and concentrated to afford **1c** (761 mg; 90%) as an off-white solid. This compound was a very potent inhibitor of tyrosinase (EC₅₀ 0.00098 mM), significantly better than **2a** (EC₅₀ 0.015 mM)(see Table 1).
¹H NMR (DMSO-d₆) δ 9.25 (br s, 1H); 8.08 (s, 1H); 6.45 (s, 1H); 4.95 (s, 2H); 2.39 (t, 2H, J = 7.42 Hz); 1.59-1.49 (m, 2H); 1.31-1.20 (m, 4H); 0.85 (m, 3H).

### Reference Example 5

### Preparation of 5-hydroxy-4H-pyran-4-on-2-methyl octanoate (Id)

Novozyme 435 (500 mg; 50 wt%) and dried 4A molecular sieves (2 g; 2 wt equiv) were added to a flask. 2-Hydroxymethyl-5-hydroxy-4H-pyran-4-one (**2a**; 1.00 g; 7.04 mmol) was added and washed in with 20 mL of acetonitrile. Octanoic acid (2.23 mL; 14.07mmol; 2 equiv) was added and the mixture was heated to 50°C overnight, at which time tle analysis (ethyl acetate eluent) indicated conversion to **1d**. The reaction mixture was filtered and the filtrate was concentrated at reduced pressure. The crude product was dissolved in ethyl acetate and washed with water (10 mL), saturated sodium bicarbonate (2x10 mL), dried with magnesium sulfate, and concentrated to afford 1.81 g of a colorless oil. This oil was dissolved in heptane and concentrated to afford a waxy solid. The solid was triturated with heptane, filtered, washed with heptane, and dried to afford 0.79 g (42%) of **1d**. This compound was a very potent inhibitor of tyrosinase (EC₅₀ 0.00039 mM), significantly better than **2a** (EC₅₀ 0.015 mM)(see Table 1).
¹H NMR (DMSO-d₆) δ 9.24 (br s, 1H); 8.07 (s, 1H); 6.45 (s, 1H); 4.95 (s, 2H); 2.390 (t, 2H, J = 7.15 Hz); 1.53 (m, 2H); 1.24 (m, 8H); 0.85 (m, 3H).

### Reference Example 6

### Preparation of 5-hydroxy-4H-pyran-4-on-2-methyl octanoate (1d) by enzymatic esterification using octanoic anhydride

2-Hydroxymethyl-5-hydroxy-4H-pyran-4-one (**2a**; 1.04 g; 7.32 mmol) was slurried in 20 mL of acetonitrile and octanoic anhydride (2.90 g; 8.05 mmol; 1.1 equiv) was added. Novozyme 435 (0.33g) was added and the mixture was stirred at ambient temperature for 12 h to completely consume **2a** according to IIPLC analysis. The reaction mixture was Filtered and the filtrate was concentrated at reduced pressure at ambient temperature. The residue was dissolved in heptane and washed with a 1:1:1 mixture of water:saturated aqueous sodium bicarbonate:methanol (3x30 mL). The organic solution was dried with magnesium sulfate and concentrated and filtered through a pad of flash silica gel and eluted with 2:1 ethyl acetate:heptane to afford 1.91 g of a mixture of **1d** and octanoic acid. The material was recrystallized from a minimum volume of hot heptane by cooling to ambient temperature to afford 1.38 g (70%) of **1d**.

### Reference Example 4'

### Preparation of 2-hydroxymethyl-4H-pyran-4-on-5-yl octanoate (6d)

2-Chloro-1-methylpyridinium iodide (1.84 g; 7.19 mmol; 1.2 equiv) and octanoic acid (0.95 mL; 6.00 mmol; 1.0 equiv) were slurried in 10 mL of dichloromethane. Triethylamine (2.01 mL; 14.39 mmol; 2.4 equiv) was added and the mixture was stirred at ambient temperature for 15 min. 2-Hydroxymethyl-5-hydroxy-4H-pyran-4-one (**2a**; 852 mg; 6.00 mmol) was added and washed in with 5 mL of dichloromethane. The mixture decolorized from a yellow to a tan slurry over ca. 30 min. The reaction mixture was stirred overnight at ambient temperature to afford one major and one minor spot by tle (3:2 ethyl acetate:heptane). This mixture was filtered and the precipitate was washed with dichloromethane. The combined filtrate was concentrated and the residue was filtered through a pad of flash silica gel and eluted with a solvent gradient of 3:2 to 4:1 ethyl acetate:heptane to afford 864 mg (54%) of **6d**. This compound was a significantly less potent inhibitor of tyrosinase (EC₅₀ 0.18 mM) than **2a** (EC₅₀ 0.015 mM)(see Table 1).
¹H NMR (DMSO-d₆) δ 8.45 (s, 1H); 6.42 (s, 1H); 5.76 (t, 1H, J - 6.05 Hz); 4.34 (d, 2H, J - 5.50 Hz); 2.54 (t, 2H, J = 7.15 Hz); 1.65-1.55 (m, 2H); 1.38-1.25 (m, 8H); 0.86 (t, 3H, J = 6.60 Hz).

### Reference Example 5'

### Preparation of 2-octanoyloxymethyl-4H-pyran-4-on-5-yl octanoate (7d)

2-Chloro-1-methylpyridinium iodide (2.16 g; 8.44 mmol; 2.4 equiv) and octanoic acid (1.28 mL; 8.44 mmol; 2.4 equiv) were slurried in 10 mL of dichloromethane. Triethylamine (2.35 mL; 16.9 mmol; 4.8 equiv) was added and the mixture was stirred at ambient temperature for 15 min. 2-Hydroxymethyl-5-hydroxy-4H-pyran-4-one (**2a**; 500 mg; 3.52 mmol) was added and washed in with 5 mL of dichloromethane. The mixture initially became an almost homogeneous yellow solution then turned into a tan slurry over ca. 30 min. The reaction mixture was stirred overnight at ambient temperature to afford a single spot by tlc (3:2 ethyl acetate:heptane). This mixture was filtered and the precipitate was washed with dichloromethane. The combined filtrate was concentrated and the residue was filtered through a pad of flash silica gel and eluted with 2:1 ethyl acetate:heptane to afford 1.07 g (83%) of **7d**. This compound was a poor inhibitor of tyrosinase (EC₅₀ >1.0 mM) (see Table 1).
¹H NMR (DMSO-d₆) δ 8.51 (s, 1H); 6.57 (s, 1H); 5.00 (s, 2H); 2.55 (t, 2H, J = 7.42 Hz); 2.41 (t, 2H, J = 7.42 Hz); 1.62-1.49 (m, 4H); 1.30-1.25 (m, 16H); 0.88-0.83 (m, 6H).

### Reference Example 7

### Preparation of 5-hydroxy-4H-pyran-4-on-2-methy/ lipoate (1e)

Novozyme 435 (400 mg; 80 wt%) and dried 4A molecular sieves (1 g; 2 wt equiv) were added to a flask. 2-Hydroxymethyl-5-hydroxy-4H-pyran-4-one (**2a**; 500 mg; 3.52 mmol) was added and washed in with 10 mL of acetonitrile. Lipoic acid (1.0 g; 4.85 mmol; 1.38 equiv) was added and the mixture was heated to 50°C overnight, at which time tlc analysis (ethyl acetate cluent) indicated conversion to **1c**. The reaction mixture was filtered and the filtrate was concentrated at reduced pressure. The crude product was dissolved in ethyl acetate and washed with saturated sodium bicarbonate (2x 10 mL), dried with magnesium sulfate, and concentrated. The crude product was filtered through a pad of flash silica gel and eluted with 4:1 ethyl acetate:heptane to afford 350 mg (30%) of **1e**. This compound was a very potent inhibitor of tyrosinase (EC₅₀ 0.00093 mM), significantly better than **2a** (EC₅₀ 0.015 mM)(sec Table 1).
¹H NMR (DMSO-d₆) δ 9.20 (br s, 1H); 8.08 (s, 1H); 6.46 (s, 1H); 4.95 (s, 2H); 3.65-3.56 (m,1II); 3.23-3.06(m, 2H); 2.46-2.35(m, 1H); 2.21 (t, 2H, J = 7.15 Hz); 1.92.1.80 (m, 1H); 1.68-1.46 (m, 4H); 1.42-1.32 (m, 2H).

### Reference Example 8

### Preparation of 4-hydroxybenzyl accetate (lf)

4-Hydroxybenzyl alcohol (2b) (3.5 g; 28.2 mmol) was slurried in 20 mL of acetonitrile. Vinyl acetate (3.64 mL; 39.5 mmol; 1.4 equiv) was added followed by Novozyme 435 (500 mg; 14 wt%). The reaction mixtures was stirred at ambient temperature for 6h, at which time tic analysis (1:1 ethyl acetate:heptane eluent) indicated no 2b and a large single non-polar spot. The reaction mixture was filtered and the filtrate was concentrated at reduced pressure to afford 4.67g (99%) of 1f. This compound was an inhibitor of tyrosinase (EC₅₀ 0.038 mM), and was more potent than 2b (EC₅₀ 0.19 mM)(see Table 1).
¹H NMR (DMSO-d₆) δ 9.6 (br s, 1H); 7.17 (m, 2H); 6.74 (m, 2H); 4.93 (s, 2H); 2.01 (s, 3H).

### Reference Example 6'

### Preparation of 4-acetoxybenzyl alcohol (6f)

4-Hydroxybenzyl alcohol (**2b**) (500 mg; 4.03 mmol) was slurried in 15 mL of dichloromethane. Triethylamine (0.84 mL; 6.04 mmol; 1.5 equiv) was added and then acetic anhydride (0.39 mL.; 4.03 mmol; 1.0 equiv) was added dropwise. The heterogeneous mixture became homogeneous over 30 min and was stirred overnight at ambient temperature to afford partial conversion to **6f** by tic analysis (1:1 ethyl acctate:heptane). The reaction mixture was concentrated and the residue was filtered through a pad of flash silica gel and eluted with 3:2 heptane:ethyl acetate to afford 390 mg (58%) of **6f**. This compound was a less potent inhibitor of tyrosinase (EC₅₀ 0.92 mM) than **2b** (EC₅₀) 0.19 mM)(sce Table 1).
¹H NMR (DMSO-d₆) δ 7.34 (d, 2H, J = 7.97 Hz); 7.06 (m, 2H); 5.215 (t, 1H, J = 5.77 Hz); 4.49 (d, 2H, J = 5.77.Hz); 2.26 (s. 3H).

### Reference Example 9

### Preparation of 4-hydroxyhenzyl propionale (1g)

4-Hydroxybenzyl alcohol (2b) (530 mg; 4.27 mmol) was dissolved in 14 mL of acetonitrile. Propionic acid (2.0 mL; 26.8 mmol; 6.3 equiv) was added and Novozyme 435 (625 mg; 1.17 wt equiv) and dried 4A molecular sieves (900 mg; 1.7 wt equiv) were added. The reaction mixture was stirred and heated to 50°C overnight, at which point HPLC analysis indicated about 30% conversion to 1g. The reaction mixture was filtered and the precipitate washed with acetonitrile and toluene. The combined filtrate/wash was concentrated and the residue was dissolved in ethyl acetate and washed with saturated sodium bicarbonate (10 mL). The organic solution was dried with magnesium sulfate, concentrated, and the residue was filtered through a pad of flash silica gel and eluted with 1:4 ethyl acctate:heptane to afford 185 mg (24%) of 1g as a colorless oil. This compound was a potent inhibitor of tyrosinasc (EC₅₀ 0.017 mM), significantly better than **2b** (EC₅₀ 0.19 mM)(see Table 1).
¹H NMR (DMSO-d₆) δ 9.50 (s, 1H); 7.17 (d, 2H, J = 8.25 Hz); 6.74 (d, 2H, J = 7.97 Hz); 4.94 (s, 2H); 2.31 (2H, q, J = 7.42 Hz); 1.02 (t, 3H, J = 7.42 Hz).

### Reference Example 10

### Preparation of 4-hydroxybenzyl propionate (1g) by enzymatic esterification with propionic anhydride

4-Hydroxybenzyl alcohol (**2b**) (5.00 g; 40.3 mmol) was dissolved in 100 mL of acctonitrile. Propionic anhydride (6.20 mL; 48.3 mmol; 1.2 equiv) was added followed by Novozyme 435 (0.25g). The mixture was stirred at ambient temperature for 18 h to almost completely convert **2b** to 1g according to IIPLC analysis. The mixture was filtered and the filtrate was concentrated at reduced pressure at ambient temperature. The residue was dissolved in ethyl acetate and washed with saturated aqueous sodium bicarbonate solution (2x25 mL). The organic solution was dried with magnesium sulfate and concentrated, and the crude product was filtered through a pad of flash silica gel to afford **1h** (5.84 g; 80%) as a colorless oil.

### Reference Example 7'

### Preparation of 4-propiony/oxyhenzyl alcohol (6g)

4-Hydroxybenzyl alcohol (**2h**) (500 mg; 4.03 mmol) was slurried in 15 mL of dichloromethane. Triethylamine (0.84 mL; 6.04 mmol; 1.5 equiv) was added and then propionic anhydride (0.52 mL; 4.03 mmol; 1.0 equiv) was added dropwise and the heterogeneous mixture became homogeneous within one minute. The reaction mixture was stirred overnight at RT to afford one major and one minor spot according to tlc analysis (1:1 ethyl acetate:heptane. The mixture was concentrated and the residue was filtered through a pad offlash silica gel and eluted with 3:2 heptane:ethyl acetate to afford 442 mg (61 %) of 6g. This compound was a less potent inhibitor of tyrosinase (EC₅₀ 1.01 mM) than **2b** (EC₅₀ 0.19 mM)(see Table 1).
¹H NMR (DMSO-d₆) δ 7.34 (m, 2H); 7.06 (m, 2H); 5.22 (br t, 1H); 4.49 (d, 2H, J = 4.94 Hz); 2.59 (2H, q, J = 7.42 Hz); 1.13 (t, 3H, J = 7.42 Hz).

### Reference Example 8'

### Preparation of 4-propionyloxybenzyl propionate (7g)

4-Hydroxybenzyl alcohol (**2b**) (500 mg; 4.03 mmol) was slurried in 15 mL of dichloromethane. Triethylamine (1.40 mL; 10.1 mmol; 2.5 equiv) was added and then propionic anhydride (1.14 mL; 8.9 mmol; 2.2 equiv) was added dropwise. The heterogeneous mixture became homogeneous over 15 min and was stirred overnight at ambient temperature to afford a single non-polar spot according to tlc analysis (1:1 ethyl acetate:heptane). The reaction mixture was concentrated and the residue was dissolved in ethyl acetate. The organic solution was washed with 1.5 M HCl (20 mL) and saturated sodium bicarbonate (10 mL) and dried with magnesium sulfate. Concentration of this organic solution afforded 923 mg (97%) of 7g. This compound was a poor inhibitor of tyrosinase (EC₅₀ > 10 mM) (see Table 1).
¹H NMR (DMSO-d₆) δ 7.40 (m, 2H); 7.12 (m, 2H); 5.08 (s, 2H); 2.60 (q, 2H, J = 7.42 Hz); 2.37 (q, 2H, J = 7.42 Hz); 1.13 (t, 3H, J = 7.42 Hz); 1.04 (t, 3H, J = 7.42 Hz).

### Reference Example 11

### Preparation (of 4-hydroxybenzyl hexanoate (1h) by enzymatic esterification with hexanoic anhydride

4-Hydroxybenzyl alcohol (**2b**) (500 mg; 4.03 mmol) was dissolved in 10 mL of acetonitrile. Hexanoic anhydride (1.12 mL; 4.83 mmol; 1.2 equiv) was added followed by Novozyme 435 (120 mg). The mixture was stirred at ambient temperature for 7.5 h to almost completely convert **2b** to **1h** according to HPLC analysis. The mixture was filtered and the filtrate was concentrated at reduced pressure at ambient temperature. The residue was dissolved in ethyl acetate and washed with saturated aqueous sodium bicarbonate solution (3x10 mL). The organic solution was dried with magnesium sulfate and concentrated to afford 1h (832 mg; 93%) as a colorless oil. This compound was a potent inhibitor of tyrosinase (EC₅₀ 0.049 mM), and was a better inhibitor than **2b** (EC₅₀ 0.19 mM)(see Table 1).
¹H NMR (DMSO-d₆) δ 9.49 (s, 1H); 7.17-7.14 (m, 2H); 6.76-6.71 (m, 2H); 4.94 (s, 2H); 2.28 (t, 2H, *J* = 7.42 Hz); 1.56-1.46 (m, 2H); 1.31-1.21 (m, 4H); 0.83 (m, 3H).

### Reference Example 12

### Preparation of 4-hydroxybenzyl octanoate (li)

4-Hydroxybenzyl alcohol (2b) (250 mg; 2.02 mmol) was dissolved in 5 mL of acetonitrile. Octanoic acid (500 uL; 3.3 mmol; 1..63 equiv) was added follewed by Novozyme 435 (200 mg; 80 wt%) and 4A molecular sieves (500 mg; 2 wt equiv). The mixture was stirred and heated to 50°C overnight. The mixture was cooled to ambient temperature, filtered, and the precipitate was washed with acetonitrile. The combined filtrate was concentrated and the residue was dissolved in ethyl acetate and washed with saturated sodium bicarbonate (2x10 mL). The organic solution was dried (magnesium sulfate) and concentrated, and the crude product was filtered through a pad of flash silica gel and cluted with 1:4 ethyl acetate:heptane to afford 960 mg of 1i which contained some residual octanoic acid.
¹H NMR (DMSO-d₆) δ 9.48 (br s, 1H); 7.18-7.13 (m, 2H); 6.75-6.71 (m, 2H); 4.94 (s, 2H); 2.28 (t, 2H, J = 7.42 Hz); 1.51 (m, 2H); 1.22 (m, 8H); 0.84 (m, 3H).

### Reference Example 13

### Preparation of 4-hydroxybenzy/ octanoate (li) by transesterification

4-Hydroxybenzyl acetate (**1f**) (83 mg; 0.50 mmol) was dissolved in 1.5 mL of toluene. Octanoic acid (158 uL; 1.0 mmol; 2.0 equiv) was added followed by Novozyme 435 (60 mg). The mixture was stirred at ambient temperature overnight, at which point HPLC analysis indicated 58.1% conversion to **1i** (40.4% **1f** and 1.4% 4-hydroxybenzyl alcohol were also observed).

### Reference Example 14

### Preparation of 4-hydroxyhenzyl octanoate (li) by transesterification in the presence of Amberlyst A-21

4-Hydroxybenzyl acetate (**1f**) (83 mg; 0.50 mmol) and dried Amberlyst A-21 (83 mg; 1 wt equiv) were combined in 1.5 mL of toluene. Octanoic acid (158 uL; 1.0 mmol; 2.0 equiv) was added followed by Novozyme 435 (60 mg). The mixture was stirred at ambient temperature overnight at which point HPLC analysis indicated 88.2% conversion to **1i** (11.1% **1f** and 0.8% 4-hydroxybenzyl alcohol were also observed).

### Reference Example 15

### Preparation of 4-hydroxybenzyl lipoate (lj)

4-Hydroxybenzyl alcohol (**2b**) (520 mg; 4.19 mmol; 1.24 equiv) was dissolved in 10 mL of acetonitrile. Lipoic acid (0.70 g; 3.39 mmol) was added followed by Novozyme 435 (150 mg) and 4A molecular sieves (1g). The mixture was stirred at ambient temperature overnight. The mixture was cooled to ambient temperature, filtered, and the precipitate was washed with acetonitrile. The combined filtrate was concentrated and the residue was dissolved in ethyl acetate and washed with saturated sodium bicarbonate (2 x 10 mL). The organic solution was dried (MgSO₄) and concentrated, and the crude product was filtered through a pad of flash silica gel and eluted with 1:4 ethyl acetate:heptane to afford 184 mg (16%) of **1j**.
¹H NMR (DMSO-d₆) δ 9.50 (s, 1H); 7.18.7.14 (m, 2H); 6.75-6.70 (m, 2H); 4.94 (s, 2H); 3.62-3.52 (m, 1H); 3.27-3.06 (m, 2H); 2.43-2.35 (m, 1H); 2.31 (t, 2H, **J** = 7.15 Hz); 1.89-1.78 (m, 1H); 1.68-1.46 (m, 4H); 1.39-1.23 (m, 2H).

### Reference Example 16

### Preparation of 2-acyloxymethyl-5-hydroxy-4H-pyran-4-one from mixed fatty acid ester without solvent (Ik)

Novozyme 435 (100 mg) and 2-hydroxymethyl-5-hydroxy-4H-pyran-4-one (**2a**; 100 mg; 0.7770 mmol) was added to a vial and 2 mL; of mixed ethyl esters from passion fruit oil was added. The mixture was heated to 60°C overnight, at which time tlc analysis (ethyl acetate eluent) indicated significant conversion to **1k**.

Tyrosinase is responsible for catalyzing the first two steps in the biosynthetic pathway leading from tyrosine to melanin. It hydroxylatcs tyrosine to dihydroxyphenylalanine (L-DOPA) and subsequently oxidizes L-DOPA to dopaquinone. Our method for determining the tyrosinase inhibition activity of various compounds focuses on the oxidation step of L-DOPA to dopaquinone by the spectrophotometric appearance of dopaquinone at 475 nm. The enzyme assay was largely based on the method described in Zhang, JT., Chen, QX., Song, KK., & Xic, JJ. Food Chemistry 2006, 95, 579-584*.*

The compounds of interest are evaluated for solubility in an aqueous environment and appropriate dilutions prepared in either water or dimethyl sulfoxide. A wide range of dilutions arc prepared from stock solutions, typically to measure final inhibitor concentrations from 10nM to 10mM.

The assay mixture is composed of 50mM Na₂HPO₄/NaH₂PO₄ pH 7.0 and 0.5mM L-DOPA. The enzymatic reaction is started by the addition of 18 Units of mushroom tyrosinase (Sigma T3824). A baseline initial rate of tyrosinase activity is measured at 475nm using a Deckman Coulter DU 800 UV/Vis Spectrophotometer in 1ml reaction format at 30° C, then a 25ul aliquot of the inhibitor solution is added/mixed and the change in rate is noted. The change in rate relates to the percent inhibition of tyrosinase due to the presence of the inhibitor. Inhibitory effects of any DMSO present are minimized by limiting the finial concentration to 2.5% and accounting for any background inhibition with DMSO blanks for each assay.

The degree of tyrosinase inhibition was measured in terms of the concentration of inhibitor necessary to inhibit tyrosinase by 50%, the EC₅₀ value. This was determined by sigmoidal dose-response curves generated in Graphpad Prizm® Version 4 by plotting the log of inhibitor concentration against the rate response (% inhibition). The data for the various examples are in Table 1 below.

**Table 1: Tyrosinase Inhhibition Values**

| **Compound** | **EC₅₀ (millimolar)** | | **Compound** | **EC₅₀ (millimolar)** |
|---|---|---|---|---|
| **2a** | 0.015 | | **2b** | 0.19 |
| **1a** | 0.0049 | | **1f** | 0.038 |
| **6a** | 0.084 | | **6f** | 0.92 |
| **1b** | 0.0046 | | **1g** | 0.017 |
| **6b** | 0.10 | | **6g** | 1.01 |
| **7b** | 0.097 | | **7g** | >10 |
| **1c** | 0.00098 | | **1h** | 0.049 |
| **1d** | 0.00039 | | | |
| **6d** | 0.18 | | | |
| **7d** | >1.0 | | | |
| **1e** | 0.00093 | | | |

## Claims

1. A cosmetic method of brightening skin comprising the application to a skin site of a composition comprising an ester compound represented by fomula 1: 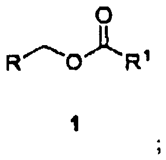 and
a cosmetically acceptable carrier
wherein R is selected from the group consisting of substituted or uusubstitutcd C₆-C₂₂ carbocyclic hydroxyaryl and
R¹ is selected from the group consisting of C₁-C₂₂ alkyl, C₂-C₂₂ alkenyl, C₄-C₂₂ dienyl, C₆-C₂₂ trienyl, C₈-C₂₂ tetraenyl and mixtures thereof.

## Patentansprüche

1. Kosmetisches Verfahren zum Aufhellen von Haut, umfassend das Auftragen einer Zusammensetzung, die eine Esterverbindung dargestellt durch Formel 1: 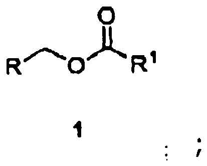 und
einen kosmetisch akzeptablen Träger umfasst, auf eine Hautstelle,
wobei R ausgewählt ist aus der Gruppe bestehend aus substituiertem oder nicht substituiertem C₆-C₂₂ carbozyklischem Hydroxyaryl und
R¹ ausgewählt ist aus der Gruppe bestehend aus C₁-C₂₂ Alkyl, C₁-C₂₂ Alkenyl, C₄-C₂₂ Dienyl, C₆-C₂₂ Trienyl, C₈-C₂₂ Tetraenyl und Mischungen davon.

## Revendications

1. Procédé cosmétique de blanchiment de la peau comprenant l'application, sur un site cutané, d'une composition comprenant un composé d'esters représenté par la formule 1 : 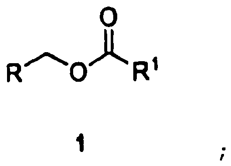 et
un excipient acceptable sur le plan cosmétique où R est choisi dans le groupe constitué par hydroxyaryle carbocyclique en C₆ à C₂₂ substitué ou non substitué et
R¹ est choisi dans le groupe constitué par alkyle en C₁ à C₂₂, alcényle en C₂ à C₂₂, diényle en C₄ à C₂₂, triényle en C₆ à C₂₂, tétraényle en C₈ à C₂₂ et leurs mélanges.
